# EUROPEAN PATENT SPECIFICATION

(11) **EP 3 266 431 B1**
(45) Date of publication and mention of the grant of the patent: **23.12.2020**
(21) Application number: 16759107.2
(22) Date of filing: 29.02.2016
(51) Int. Cl.: A61F 13/42, A61F 13/44, A61B 5/20

(54) **PORTABLE EXCREMENT DETECTION TERMINAL**
TRAGBARES EXKREMENTENDETEKTIONSENDGERÄT
TERMINAL PORTABLE DE DÉTECTION D'EXCRÉMENTS

(30) Priority: 02.03.2015 KR 20150029339; 29.01.2016 KR 20160011620
(43) Date of publication of application: 10.01.2018
(73) Proprietor: Dtron Co., Ltd., Incheon 22013 (KR)
(72) Inventor: AN, Sung Hoon, Bucheon-si Gyeonggi-do 14548 (KR)
(74) Representative: Müller Verweyen
(86) International application number: PCT/KR2016/001975
(87) International publication number: WO 2016/140471

(56) References cited:
- EP-A2- 2 579 069
- WO-A1-2013/091707
- KR-A- 20010 037 116
- KR-A- 20020 082 593
- KR-A- 20110 034 751
- KR-A- 20110 050 282
- KR-B1- 101 596 425
- US-A- 5 760 694
- US-A1- 2012 220 969

## Description

### [Technical Field]

The present invention relates to a portable excrement detection terminal, and more particularly, to a portable excrement detection terminal which detects the excrement from the outside of clothes without making contact with a human body, in which a low radio frequency (RF) is used to accurately determine the presence of excrement regardless of the thickness of clothes and with the minimized influence of an error caused by the human body.

### [Background Art]

In general, people who are not aware of defecation/urination or who are unable to handle the defecation/urination themselves, such as infants, senior citizens with dementia, and patients with a serious illness who have difficulties in behavior, face a situation in which they need help of a guardian or caregiver to deal with their feces and urine. Therefore, the guardian or caregiver deals with the feces and urine by putting a diaper or undergarment on the infants, the senior citizens with dementia, the patients with a serious illness or the like, and frequently checking a defecation/urination state to change the diaper or undergarment.

In this case, there is an inconvenience in that the guardian or caregiver has to visually and frequently check the defecation and urination state of the infants, the senior citizens with dementia, the patients with a serious illness or the like in order to check whether there is the feces and urine. In addition, in the case of managing a large number of patients in a patient's room or the like, the guardian or caregiver will face difficulties in the management that requires to periodically check a large number of patients. Particularly, in the case of periodically managing a large number of patients, it is difficult to directly check the condition of the diaper by taking off the patient's clothes one by one.

In order to reduce some of such inconvenience, there has been proposed a technique of attaching a urine detection sensor to the diaper (or the garment), detecting urine by using the urine detection sensor, and transmitting the result of detection to a terminal. At this time, an RF scheme is mainly used to transmit a urine detection signal to the terminal.

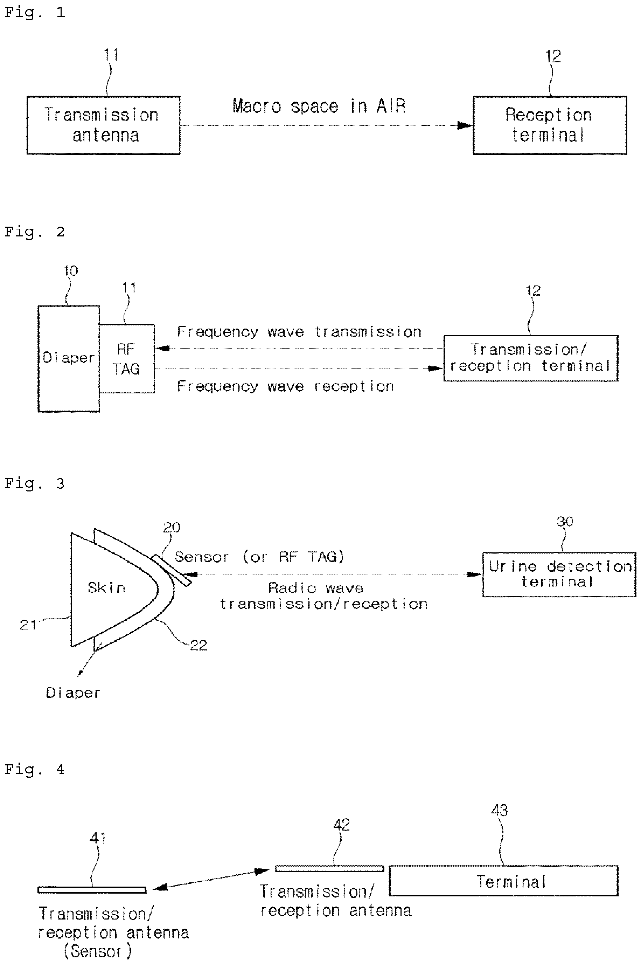

FIGS. 1 and 2 show one example of a urine detection apparatus using an RF according to the related art.

FIGS. 1 and 2 show the RF technology in which a transmission antenna (or an RF tag) 11 serving as a sensor is attached to a diaper 10 in a macroscopic space (or environment), a reception terminal (or a transmission/reception terminal) 12 radiates an RF signal to the transmission antenna 11, and a urine detection state is determined according to a reception state of the RF signal returned from the transmission antenna 11. At this time, the transmission antenna (or the RF tag) 11 generates a urine detection signal (frequency wave) based on that signal quantities are changed (signals are relatively small when there is urine) as a radio wave (frequency wave) is absorbed by a medium due to the urine (or feces). In this case, if interference is caused by a medium that affects a human body and surrounding frequency waves, it is difficult to detect the urine, and the probability of error occurrence is very high.

The technique using the RF as described above uses a high-frequency wave to transmit a frequency wave in a macroscopic space.

Meanwhile, FIG. 3 shows another example of a urine detection apparatus using an RF according to the related art. A urine detection sensor (or an RF tag) 20 for urine detection is mounted on a diaper 22 worn on skin 21. A urine detection signal obtained by detecting the urine by the urine detection sensor 20 is transmitted to a urine detection terminal 30. At this time, the urine detection signal generated by the urine detection sensor 20 is accurately transmitted to the urine detection terminal 30 in a space without an obstacle. However, if clothes are put on a diaper 30, radio wave interception occurs, so that the urine detection signal cannot be accurately transmitted to the urine detection terminal 30.

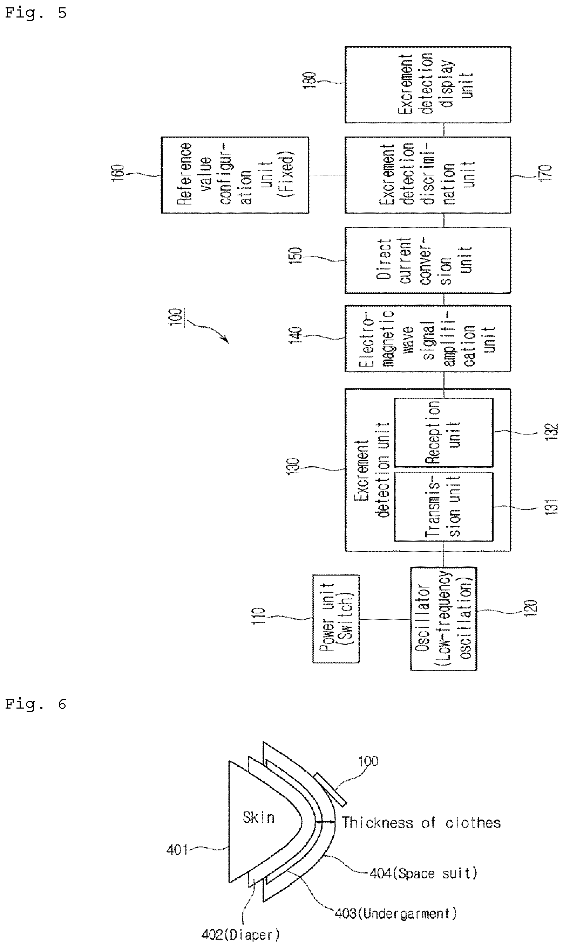

In addition, FIG. 5 shows another embodiment of a urine detection apparatus according to the related art, in which a sensor and a urine detection terminal are separated from each other. A sensor 41 for detecting the urine and a terminal 43 for detecting the urine and displaying a result of the detection are separated from each other. The terminal 43 is provided with a transmission/reception antenna 42 for wirelessly receiving a signal from the sensor 41. Since the urine detection apparatus having such a configuration requires attaching the sensor 41 to a detection target one by one, it is inconvenient to use.

Meanwhile, according to the related art, another technique for detecting urine in a non-contact manner is disclosed in Korean Patent Registration No. 10-1260997 (registered on April 29, 2013, and hereinafter referred to as "Patent document 1") and Korean Patent Application Publication No. 10-2009-0081886 (published on July 29, 2009, and hereinafter referred to as "Patent document 2").

The related art disclosed in Patent document 1 provides a feces and urine detector in which a plurality of capacitive electrodes are formed on a thin film insulation member having flexibility, so that a feces and urine state and the number of urination and defecation can be obtained, and it can be determined whether there is urine or feces. In addition, a capacitive feces and urine detector having a plurality of electrodes to automatically detect the feces and urine state and to discriminate the urine and the feces is provided for those who cannot deal with urination themselves.

In addition, according to the Patent document 2, a diaper excrement detection apparatus using a capacitive sensor includes: a detection unit including the capacitance sensor for sensing a capacitance change due to urine absorbed in a diaper in a non-contact manner; an output unit that operates if a detection signal from the detection unit exists; a power supply unit for supplying energy required for operating the detection unit and the output unit; and a control unit electrically connected to the detection unit/output unit/power supply unit to control operation of each unit. The diaper excrement detection apparatus automatically detects excretion of the feces and urine by monitoring, in a non-contact manner, the inside of the diaper worn on an infant who is not toilet-trained, and a senior and infirm citizen or serious patient who is not free in behavior. Then, a buzzer, a melody, an LED or a vibration is used to inform the guardian of an excrement state through a wired/wireless output.

EP 2 579 069 A2, WO 2013/091707 A1 and US 2012/220969 A1 each disclose a portable excrement detection terminal comprising an oscillator, an excrement detection unit, an electromagnetic wave signal amplification unit, a direct current conversion unit, an excrement detection discrimination unit and an excrement detection display unit.

### [Disclosure]

### [Technical Problem]

Although the above techniques of the related art can detect the excrement by using a capacitive scheme or in a non-contact manner, it is inconvenient to actually attach a sensor for detecting the excrement in a non-contact manner to the diaper.

In addition, the related art disclosed in Patent document 1 uses a plurality of electrodes for detecting the feces and urine, so that a sensor configuration cost is high, and resources are largely wasted because the sensor is discarded when the diaper is replaced due to the detection of the feces and urine.

In addition, since the related art uses a high-frequency wave to transmit a frequency wave in a macroscopic space, there is a disadvantage in that an error occurs depending on the thickness of clothes due to the influence of the human body in a microscopic space.

Therefore, the present invention has been proposed to solve the problems occurring in the related art as described above, and an object of the present invention is to provide a portable excrement detection terminal which detects the excrement (feces and urine) without making contact with a human body, in which a low radio frequency (RF) is used to accurately determine the presence of excrement regardless of the thickness of clothes and with the minimized influence of an error caused by the human body.

Another object of the present invention is to provide a portable excrement detection terminal in which an excrement detection apparatus is implemented in a portable terminal where a sensor and a detection terminal are integrated with each other, so that convenience of use is improved.

Still another object of the present invention is to provide a portable excrement detection terminal in which the low-frequency wave, which is transmitted from the transmission unit, is transmitted to the reception unit through the excrement serving as a medium, so that the excrement is accurately detected.

### [Technical Solution]

To achieve the objects described above, according to the present invention, there is provided a portable excrement detection terminal including: an oscillator for generating a low-frequency wave; an excrement detection unit for radiating the low-frequency wave generated from the oscillator to a measurement target, and detecting excrement by taking the excrement as a medium; an electromagnetic wave signal amplification unit for amplifying an electromagnetic wave signal outputted from the excrement detection unit; a direct current conversion unit for converting an excrement detection signal amplified by the electromagnetic wave signal amplification unit into a direct current; an excrement detection discrimination unit for comparing the direct current obtained from the direct current conversion unit, with a preset reference value, and outputting a result of the comparison as an excrement detection discrimination value; and an excrement detection display unit for visually displaying detection of the excrement based on the excrement detection discrimination value outputted from the excrement detection discrimination unit, wherein the excrement detection unit includes: a transmission unit mounted on a bottom surface of a printed circuit board (PCB) on which an excrement detection component is mounted, to transmit the low-frequency wave; and a reception unit mounted on the bottom surface of the printed circuit board to receive the low-frequency wave, which is transmitted from the transmission unit, as the excrement detection signal, if the low-frequency wave is received through the excrement serving as a medium, wherein the excrement detection component is mounted on a top surface of the printed circuit board to operate as a ground (GND).

In detail, the transmission unit and the reception unit may be spaced apart from each other by a predetermined distance about the ground.

In detail, in the excrement detection unit, if the excrement is not detected, the low-frequency wave transmitted from the transmission unit may be weakly transmitted to the reception unit.

In detail, the excrement detection unit may radiate a wave having a frequency within a range that does not affect a human body to detect the excrement.

In addition, according to the present invention, there is provided a detection method using a portable excrement detection terminal including: (a) outputting a reference frequency wave and a sensor frequency wave for detecting feces and urine when an operation signal for detecting the feces and urine is generated; (b) detecting a frequency wave that is changed by a measurement target; (c) generating a detection signal by logically computing the reference frequency wave and the detected frequency wave; (d) comparing the generated detection signal with a reference signal set for determining a feces and urine state; and (e) determining the feces and urine state based on a result of the comparison in step (d), and displaying a result of the determination for the feces and urine state.

### [Advantageous Effects]

According to the present invention, the excrement (feces and urine) is detected without making contact with a human body while using the low RF and using the excrement as a medium, so that the presence of excrement can be accurately determined regardless of the thickness of clothes and with the minimized influence of an error caused by the human body.

In addition, according to the present invention, an excrement detection apparatus is implemented in a portable terminal where a sensor and a detection terminal are integrated with each other, so that convenience of use can be improved.

### [Description of Drawings]

FIG. 1 is a view illustrating one example of a urine detection apparatus that detects urine by using an RF technique according to the related art.
FIG. 2 is a view illustrating another example of a urine detection apparatus that detects urine by using an RF technique according to the related art.
FIG. 3 is a view for explaining a mounting position of a urine detection sensor according to the related art.
FIG. 4 is a view illustrating a configuration of a urine detection sensor and a detection terminal according to the related art.
FIG. 5 is a block diagram showing a portable excrement detection apparatus according to the present invention.
FIG. 6 is a view illustrating a configuration of an excrement detection unit applied to the present invention.
FIG. 7 is a view illustrating detection of excrement in a portable excrement detection apparatus according to the present invention.

### [Best Mode]

### [Mode for Invention]

Hereinafter, a portable excrement detection terminal according to a preferred embodiment of the present invention will be described in detail with reference to accompanying drawings.

FIG. 5 is a block diagram showing a portable excrement detection terminal 100 according to a preferred embodiment of the present invention.

According to the present invention, a portable excrement detection terminal 100 includes a power unit 110, an oscillator 120, an excrement detection unit 130, an electromagnetic wave signal amplification unit 140, a direct current conversion unit 150, a reference value configuration unit 160, an excrement detection discrimination unit 170, and an excrement detection display unit 180.

The power unit 110 supplies a driving power with a power of an internal battery 111.

The oscillator 120 oscillates with the power supplied through the power unit 110 to output an oscillation frequency wave (electromagnetic wave) . To this end, it is preferred that the oscillator 120 generates a low-frequency wave, that is, a wave having a frequency within a range that does not affect a human body.

The excrement detection unit 130 is a sensor for actually detecting the excrement. The excrement detection unit 130 radiates the low-frequency wave generated from the oscillator 120 to a measurement target, and detects the excrement by taking the excrement as a medium.

As shown in FIG. 6, such an excrement detection unit 130 includes: a transmission unit 131 mounted on a bottom surface of a printed circuit board (PCB) 200 on which an excrement detection component 300 is mounted, to transmit the low-frequency wave; and a reception unit 132 mounted on the bottom surface of the printed circuit board 200 to receive the low-frequency wave, which is transmitted from the transmission unit 131, as an excrement detection signal, if the low-frequency wave is received through the excrement serving as a medium. At this time, the excrement detection component 300 is mounted on a top surface of the printed circuit board 200 to operate as a ground (GND), and includes electronic devices such as an amplifier, a comparator, and a displayer.

It is preferred that the transmission unit 131 and the reception unit 132 are spaced apart from each other by a predetermined distance about the ground (GND).

In the excrement detection unit 130, if the excrement is not detected, the low-frequency wave transmitted from the transmission unit 131 is weakly transmitted to the reception unit 132, so that the excrement cannot be detected by the reception unit 132. It is preferred to radiate a wave having a frequency within a range that does not affect the human body for the excrement detection. Accordingly, the present invention does not affect the human body.

In FIG. 5, the electromagnetic wave signal amplification unit 140 amplifies the electromagnetic wave signal outputted from the excrement detection unit 130.

The direct current conversion unit 150 converts an excrement detection signal amplified by the electromagnetic wave signal amplification unit 140 into a direct current.

The excrement detection discrimination unit 170 compares the direct current obtained from the direct current conversion unit 150 with a preset reference value, and outputs a result of the comparison as an excrement detection discrimination value.

The excrement detection display unit 180 visually displays detection of the excrement based on the excrement detection discrimination value outputted from the excrement detection discrimination unit 170.

The operation of the portable excrement detection terminal 100 configured as described above according to the present invention will be described in detail with reference to FIGS. 5 to 7.

First, in order to detect the excrement by using the portable excrement detection terminal 100 according to the present invention, a power switch is turned on. When the power switch is turned on, the internal battery inside the power unit 110 is discharged, and a discharge voltage is supplied to each part of the terminal through the power switch as the driving power (3V). In addition, the battery can be a rechargeable secondary battery or a primary battery that can be easily purchased on the market.

When the driving power is supplied, the oscillator 120 oscillates with the supplied power and generates an oscillation frequency wave (electromagnetic wave). It is preferred that the generated oscillation frequency wave is a low-frequency wave having a frequency within a range that does not affect a human body. By using the low-frequency wave that does not affect the human body, an error caused by the human body is minimized during the excrement detection. In other words, the low RF technique is applied to a microscopic space which is close to a diaper so as to be usefully utilized for the excrement detection in a microscopic space. If a high-frequency wave is used as in the related art, an error occurs in the excrement detection depending on the thickness of clothes due to the influence of the human body in a microscopic space. For example, in case of a very thin garment such as a diaper, it is possible to detect a certain degree of excrement. However, when undergarments or outer garments are worn on the diaper, a noise is introduced into the high-frequency wave due to the influence of the human body. The present invention uses a low-frequency wave in a microscopic space in order to overcome such disadvantages of the related art.

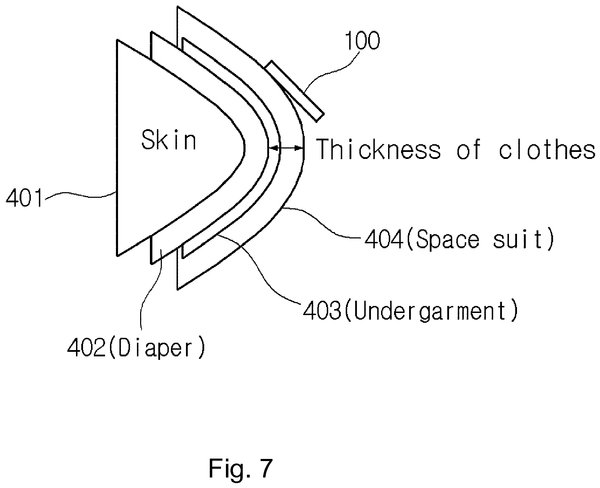

The transmission unit 131 of the excrement detection unit 130 radiates the low-frequency wave generated by the oscillator 120 to the measurement target. For example, as shown in FIG. 7, assuming that a measurement subject wears a diaper 402, wears an undergarment 403 on the diaper 402, and wears an outer garment (for example, a space suit) 404 on the undergarment 403, the excrement detection can be performed by only making the portable excrement detection terminal 100 to softly touch the space suit 404.

In addition, another feature of the present invention is that the excrement detection unit 130, which is a sensor for detecting the excrement, and the detection terminal for detecting and discriminating the excrement and displaying a result are integrated into a single article. Accordingly, it is unnecessary to separately attach a sensor for the excrement detection to the measurement target. In addition, if a user only softly makes a measurement portion of the portable excrement detection terminal 100 to touch the measurement target upon use, the detection, discrimination, displaying and the like of the excrement can be performed in a single terminal through a series of processes.

The low-frequency wave radiated from the transmission unit 131 is detected through the reception unit 132. At this time, the low-frequency wave radiated from the transmission unit 131 is transmitted to the reception unit 132 by taking the excrement as a medium. Unlike high-frequency waves, it is difficult for the low-frequency wave to move to another space without a medium because of their characteristics. Therefore, if there is no excrement, there is no medium, so that it is difficult for the low-frequency wave radiated from the transmission unit 131 to be transmitted to the reception unit 132 (the low-frequency wave can be very weakly transmitted to the reception unit 132). Otherwise, if excrement exists, the excrement serves as a medium, so that the low-frequency wave radiated from the transmission unit 131 can be transmitted to the reception unit 132 through the excrement serving as the medium. Accordingly, the reception unit 132 can accurately detect the excrement.

FIG. 6 is a view showing a configuration of the excrement detection unit 130, which is the excrement detection sensor.

In this case, the transmission unit 131 of the excrement detection unit 130 is mounted on a bottom surface of a printed circuit board 200 on which the excrement detection component 300 is mounted, and the reception unit 132 is also mounted on the bottom surface of the printed circuit board 200. At this time, the excrement detection component 300 is mounted on the top surface of the printed circuit board 200 to operate as a ground (GND).

In this case, it is preferred that the transmission unit 131 and the reception unit 132 are spaced apart from each other by a predetermined distance about the ground (GND).

Next, the electromagnetic wave signal amplification unit 140 amplifies and outputs the electromagnetic wave signal (excrement detection signal) outputted from the excrement detection unit 130 according to a preset amplification degree. The outputted excrement detection signal is transmitted to the direct current conversion unit 150.

The direct current conversion unit 150 converts the excrement detection signal amplified by the electromagnetic wave signal amplification unit 140 into a direct current. The outputted direct current voltage (excrement detection signal) is transmitted to the excrement detection discrimination unit 170.

The excrement detection discrimination unit 170 compares the direct current voltage obtained from the direct current conversion unit 150 with a reference value set through a reference value configuration unit 160, and outputs a result of the comparison as an excrement detection discrimination value.

For example, the excrement detection discrimination unit 170 compares the reference value set by the reference value configuration unit 160 with the excrement detection signal (direct current voltage) outputted from the direct current conversion unit 150, and outputs a differential value thereof as the excrement detection discrimination value.

Next, the excrement detection display unit 180 visually displays detection of the excrement based on the excrement detection discrimination value outputted from the excrement detection discrimination unit 170.

For example, the excrement detection display unit 180 visually displays the excrement detection state by using an output signal of the excrement detection discrimination unit 170. In other words, only when the excrement detection signal is generated, an output of the excrement detection discrimination unit 170 is generated, and the output is used to make a light emitting diode (LED), which is a visual display device, emit light.

Therefore, the user turns on the power source of the portable excrement detection terminal 100, makes a detection portion to touch the measurement target, and determines the excrement state by checking whether the light emitting diode (LED) is lit or not.

In this case, according to the present invention, the light emitting diode for visually displaying a power supply state is used, so that when the power is supplied, the light emitting diode is lit to allow the user to recognize that the detection terminal is normally operating at present.

Thus, the user can easily recognize the power supply state or the operation state of the portable excrement detection terminal 100.

In other words, according to the present invention, a low RF technique is applied to a microscopic space which is close to the diaper, and the excrement is detected in a manner that a low-frequency wave generated by the transmission unit is transmitted to the reception unit by taking the excrement as a medium, so that the excrement can be accurately detected regardless of the thickness of clothes.

In addition, according to the present invention, a detection method using the portable excrement detection terminal can be performed as follows.

First, a reference frequency wave and a sensor frequency wave for detecting excrement are outputted when an operation signal for excrement detection is generated, and a frequency wave that is changed by a measurement target is detected. Then, a detection signal is generated by logically computing the reference frequency wave and the detected frequency wave, and the generated detection signal is compared with a reference signal set for determining an excrement state. The excrement state is determined based on a result of the comparison, and a result of the excrement state determination is displayed.

### [Industrial Applicability]

The present invention may be effectively applied to a technique of easily detecting excrement in a state of wearing clothes by using a portable detection terminal in which a sensor and a detection terminal are integrated with each other.

## Claims

1. A portable excrement detection terminal comprising:
an oscillator for generating a low-frequency wave;
an excrement detection unit for radiating the low-frequency wave generated from the oscillator to a measurement target, and detecting excrement by taking the excrement as a medium;
an electromagnetic wave signal amplification unit for amplifying an electromagnetic wave signal outputted from the excrement detection unit;
a direct current conversion unit for converting an excrement detection signal amplified by the electromagnetic wave signal amplification unit into a direct current;
an excrement detection discrimination unit for comparing the direct current obtained from the direct current conversion unit with a preset reference value, and outputting a result of the comparison as an excrement detection discrimination value;
an excrement detection display unit for visually displaying detection of the excrement based on the excrement detection discrimination value outputted from the excrement detection discrimination unit; and
wherein the excrement detection unit includes:
a transmission unit mounted on a bottom surface of a printed circuit board (PCB) on which an excrement detection component is mounted, to transmit the low-frequency wave; and
a reception unit mounted on the bottom surface of the printed circuit board to receive the low-frequency wave, which is transmitted from the transmission unit, as the excrement detection signal, if the low-frequency wave is received through the excrement serving as a medium,
wherein the excrement detection component is mounted on a top surface of the printed circuit board to operate as a ground (GND).

2. The portable excrement detection terminal of claim 1, wherein the transmission unit and the reception unit are spaced apart from each other by a predetermined distance about the ground.

3. The portable excrement detection terminal of claim 1, wherein the excrement detection unit radiates a wave having a frequency within a range that does not affect a human body to detect the excrement.

4. The portable excrement detection terminal of claim 1, further comprising a reference value configuration unit for setting a reference value for excrement discrimination, wherein the excrement detection discrimination unit includes a comparator for comparing the reference value set by the reference value configuration unit with the excrement detection signal outputted from the direct current conversion unit, and outputting a differential value thereof as the excrement detection discrimination value.

5. The portable excrement detection terminal of claim 1, wherein the excrement detection display unit visually displays an excrement detection state based on an output signal of the excrement detection discrimination unit.

## Patentansprüche

1. Ein tragbares Exkrementdetektionsterminal, umfassend:
einen Oszillator zur Erzeugung einer Niederfrequenzwelle;
eine Exkrementdetektionseinheit zum Abstrahlen der von dem Oszillator erzeugten Niederfrequenzwelle auf ein Messziel und zum Detektieren von Exkrement durch Erfassen des Exkrements als Medium;
eine elektromagnetische Wellensignal-Verstärkungseinheit zum Verstärken eines elektromagnetischen Wellensignals, das von der Exkrementdetektionseinheit ausgegeben wird;
eine Gleichstrom-Umwandlungseinheit zum Umwandeln eines Exkrementdetektionssignals, das durch die elektromagnetische Wellensignal-Verstärkungseinheit verstärkt wird, in einen Gleichstrom;
eine Exkrementdetektions-Unterscheidungseinheit zum Vergleichen des von der Gleichstrom-Umwandlungseinheit erhaltenen Gleichstroms mit einem voreingestellten Referenzwert und zum Ausgeben eines Ergebnisses des Vergleichs als einen Exkrementdetektions-Unterscheidungswert;
eine Exkrementdetektions-Anzeigeeinheit zur visuellen Anzeige der Detektion des Exkrements auf der Grundlage des von der Exkrementdetektions-Unterscheidungseinheit ausgegebenen Exkrementdetektions-Unterscheidungswertes; und wobei die Exkrementdetektionseinheit umfasst:
eine Übertragungseinheit, die auf einer Unterseite einer gedruckten Leiterplatte (PCB) montiert ist, auf der eine Exkrementdetektionskomponente montiert ist, um die Niederfrequenzwelle zu übertragen; und
eine auf der Unterseite der Leiterplatte montierte Empfangseinheit zum Empfang der von der Übertragungseinheit übertragenen Niederfrequenzwelle als Exkrementdetektionssignal, wenn die Niederfrequenzwelle durch das als Medium dienenden Exkrement empfangen wird,
wobei die Exkrementdetektionskomponente auf einer Oberseite der Leiterplatte montiert ist, um als Masse (GND) zu dienen.

2. Das tragbare Exkrementdetektionsterminal nach Anspruch 1, wobei die Übertragungseinheit und die Empfangseinheit um eine vordefinierte Entfernung voneinander um die Masse herum beabstandet sind.

3. Das tragbare Exkrementdetektionsterminal nach Anspruch 1, wobei die Exkrementdetektionseinheit eine Welle mit einer Frequenz innerhalb eines Bereichs ausstrahlt, der einen menschlichen Körper nicht beeinträchtigt, um das Exkrement zu detektieren.

4. Das tragbare Exkrementdetektionsterminal nach Anspruch 1, das ferner eine Referenzwert-Konfigurationseinheit zur Einstellung eines Referenzwertes für die Exkrementunterscheidung umfasst,
wobei die Exkrementdetektions-Unterscheidungseinheit einen Komparator zum Vergleichen des durch die Referenzwert-Konfigurationseinheit eingestellten Referenzwertes mit dem von der Gleichstrom-Umwandlungseinheit ausgegebenen Exkrementdetektionssignal und zum Ausgeben eines Differenzwertes davon als den Exkrementdetektions-Unterscheidungswert aufweist.

5. Das tragbares Exkrementdetektionsterminal nach Anspruch 1, wobei die Exkrementdetektions-Anzeigeeinheit visuell einen Exkrementdetektionszustand auf der Grundlage eines Ausgangssignals der Exkrementdetektions-Unterscheidungseinheit anzeigt.

## Revendications

1. Un terminal portable de détection d'excréments comprenant :
un oscillateur pour générer une onde basse fréquence ;
une unité de détection d'excréments pour émettre l'onde basse fréquence générée depuis l'oscillateur vers une cible de mesure, et détecter des excréments en prenant les excréments en tant que support ;
une unité d'amplification de signal d'onde électromagnétique pour amplifier un signal d'onde électromagnétique délivré en sortie depuis l'unité de détection d'excréments ;
une unité de conversion de courant continu pour convertir un signal de détection d'excréments amplifié par l'unité d'amplification de signal d'onde électromagnétique en un courant continu ;
une unité de discrimination de détection d'excréments pour comparer le courant continu obtenu depuis l'unité de conversion de courant continu avec une valeur de référence préétablie, et délivrer en sortie un résultat de la comparaison en tant que valeur de discrimination de détection d'excréments ;
une unité d'affichage de détection d'excréments pour afficher visuellement une détection des excréments d'après la valeur de discrimination de détection d'excréments délivrée en sortie depuis l'unité de discrimination de détection d'excréments ; et
dans lequel l'unité de détection d'excréments comporte :
une unité de transmission montée sur une surface de dessous d'une carte de circuit imprimé (PCB) sur laquelle un composant de détection d'excréments est monté, pour transmettre l'onde basse fréquence ; et
une unité de réception montée sur la surface de dessous de la carte de circuit imprimé pour recevoir l'onde basse fréquence, qui est transmise depuis l'unité de transmission, en tant que signal de détection d'excréments, si l'onde basse fréquence est reçue par le biais des excréments servant de support,
dans lequel le composant de détection d'excréments est monté sur une surface de dessus de la carte de circuit imprimé pour fonctionner en tant que masse (GND).

2. Le terminal portable de détection d'excréments selon la revendication 1, dans lequel l'unité de transmission et l'unité de réception sont espacées l'une de l'autre d'une distance prédéterminée autour de la masse.

3. Le terminal portable de détection d'excréments selon la revendication 1, dans lequel l'unité de détection d'excréments émet une onde ayant une fréquence dans une plage qui n'affecte pas un corps humain pour détecter les excréments.

4. Le terminal portable de détection d'excréments selon la revendication 1, comprenant en outre une unité de configuration de valeur de référence pour établir une valeur de référence pour une discrimination d'excréments, dans lequel l'unité de discrimination de détection d'excréments comporte un comparateur pour comparer la valeur de référence établie par l'unité de configuration de valeur de référence avec le signal de détection d'excréments délivré en sortie depuis l'unité de conversion de courant continu, et délivrer en sortie une valeur différentielle de celui-ci en tant que valeur de discrimination de détection d'excréments.

5. Le terminal portable de détection d'excréments selon la revendication 1, dans lequel l'unité d'affichage de détection d'excréments affiche visuellement un état de détection d'excréments d'après un signal de sortie de l'unité de discrimination de détection d'excréments.
